# EUROPÄISCHE PATENTSCHRIFT

(11) **EP 1 427 453 B1**
(45) Veröffentlichungstag und Bekanntmachung des Hinweises auf die Patenterteilung: **05.04.2006**
(21) Anmeldenummer: 02767490.2
(22) Anmeldetag: 18.09.2002
(51) Int. Cl.: A61L 15/58, A61L 15/46, A61L 15/18, C09J 9/00, C09J 11/04, C08K 3/08

(54) **ANTIMIKROBIELLER KLEB- UND BESCHICHTUNGSSTOFF UND VERFAHREN ZUR HERSTELLUNG DESSELBEN**
ANTIMICROBIAL ADHESIVE AND COATING SUBSTANCE AND METHOD FOR THE PRODUCTION THEREOF
MATERIAU D'ADHESION ET DE REVETEMENT ANTIMICROBIEN ET SON PROCEDE DE PRODUCTION

(30) Priorität: 18.09.2001 DE 10146050
(43) Veröffentlichungstag der Anmeldung: 16.06.2004
(73) Patentinhaber: Bio-Gate Bioinnovative Materials GmbH, 90411 Nürnberg (DE)
(72) Erfinder: WAGENER, Michael, 28355 Bremen (DE); HARTWIG, Andreas, 27721 Ritterhude (DE)
(74) Vertreter: Gassner, Wolfgang
(86) Internationale Anmeldenummer: PCT/EP2002/010463
(87) Internationale Veröffentlichungsnummer: WO 2003/024494

(56) Entgegenhaltungen:
- DE-A- 19 756 790
- US-A- 4 849 223
- DATABASE WPI Section Ch, Week 199909 Derwent Publications Ltd., London, GB; Class A82, AN 1999-101274 XP002226346 & JP 10 330654 A (SUMITOMO CEMENT CO LTD), 15. Dezember 1998 (1998-12-15)
- DATABASE WPI Section Ch, Week 200171 Derwent Publications Ltd., London, GB; Class A96, AN 2001-613102 XP002226347 & JP 2001 137279 A (SEKISUI CHEM IND CO LTD), 22. Mai 2001 (2001-05-22)

## Beschreibung

Die Erfindung betrifft einen antimikrobiellen Kleb- und Beschichtungsstoff, eine Verwendung des Kleb- und Beschichtungsstoffs und ein Verfahren zu dessen Herstellung.

Die Erfindung betrifft das Gebiet der Kleb- und Beschichtungsstoffe für im weitesten Sinne medizinische, medizintechnische oder hygienetechnische Anwendungen. Insbesondere zur Herstellung von Pflastern, Wundauflagen, Kathetern, hygienischen Verpackungsmitteln, zur Beschichtung der vorgenannten Materialien sowie zur Beschichtung von Komponenten medizintechnischer Vorrichtungen, von Wänden von hygienisch zu haltenden Räumen und dgl. werden antimikrobielle Kleb- und Beschichtungsstoffe verwendet. Durch die Verwendung solcher Kleb- und Beschichtungsstoffe soll die Ansiedlung von Mikroorganismen verhindert werden.

Aus der DE 199 58 458 A1 ist eine antimikrobielle Wundauflage bekannt. Die Wundauflage ist aus einem synthetischen Polymermaterial hergestellt, welches metallionenhaltige Zeolithe enthält.

Die US 6,124,374 beschreibt eine antimikrobielle Haftcreme für Gebisse. Als antimikrobieller Wirkstoff sind hier 2-wertige Kupfersalze zugesetzt.

Aus der US 6,216,699 B1 ist ein Haftklebstoff bekannt, dem als antimikrobieller Wirkstoff Diidomethyl-p-tolylsulfon zugesetzt ist.

Die bekannten antimikrobiellen Zusätze sind nicht besonders universell. Sie sind spezifisch auf die jeweilige Matrix abgestimmt. Die antimikrobielle Wirksamkeit der damit versetzten Materialien hält nicht besonders lange an.

Aus der WO 95/20878 ist ein Verfahren zur Herstellung von bakteriziden oder fungiziden Kunststoffkörpern bekannt. Dabei wird mittels Dünnschichttechnik zunächst eine dünne Silberschicht auf eine Folie aufgebracht. Die Folie wird anschließend zerkleinert. Die zerkleinerte Folie wird aufgeschmolzen und dann mittels herkömmlicher Techniken in die gewünschte Form gebracht. - Das Verfahren ist äußerst zeit- und kostenaufwändig. Es ist die Herstellung eines besonderen Zwischenprodukts und dessen Zerkleinerung erforderlich.

Aus der EP 0 190 504 ist eine antimikrobielle Zusammensetzung bekannt, welche 5 bis 10 Gew.% Silber enthält. Zur Verbesserung der antimikrobiellen Eigenschaften ist zusätzlich ein hydratisierbares oder ein hydratisiertes Oxid zugesetzt.

Die DE 31 10 681 C2 beschreibt ein Material für Knochenimplantate. Das Material ist aus einem Polymer hergestellt, dem als antimikrobieller Wirkstoff Silberphosphat zugesetzt ist.

Aus der WO 81/02667 ist ein antimikrobielles chirurgisches Implantat bekannt. Dem Implantat ist als antimikrobieller Wirkstoff metallisches Silber zugesetzt.

Die gattungsgemäße WO 82/01990 beschreibt einen Knochenzement auf der Basis von Polymethylmethacrylat als Hauptkomponente, dem als antimikrobieller Wirkstoff 5 Vol.% eines Silbersalzes zugesetzt ist.

Die US 5,837,275 offenbart ein antimikrobielles Material, das u.a. Silberpartikel mit einer Korngröße von weniger als 200 nm enthält. Das Silbergitter weist künstlich erzeugte Gitterstörungen und Fehlstellen auf, um die Freisetzung von Silber-Ionen zu erleichtern.

Aus der WO 34/01721 ist ein mit Silbersulfat oder Silberazetat versehenes Material bekannt. Dieses Material setzt in einer umgebenden Flüssigkeit innerhalb von 24 Stunden eine Konzentration von mehr als 1 µM an Silber-Ionen frei.

Die DE 32 288 B49 A1 beschreibt ein Material mit einem Überzug aus Silber. Dem Material ist elementarer Kohlenstoff oder Titan zugesetzt. Der Zusatz soll eine erhöhte Freisetzung von Silber-Ionen in die Umgebung erleichtern.

Die US 4,849,233 offenbart einen Knochenzement, dem etwa 10 Gew,% elementares Silber sowie Titanoxid oder Tantaloxid zugesetzt sind. Der Knochenzement zeichnet sich durch eine hohe Rate der Freisetzung an Silber-Ionen aus.

Aus dem Dokument WPI/DERWENT-Zusammenfassung 1999-101274 (JP_A-10330654; SUMITOMO CEMENT CO LTD) vom 15.12.1998 ist eine antimikrobielle Beschichtung bekannt, welche in einem Harz Silber-Partikel mit einem Durchmesser von bis zu 50 nm enthält.

Das Dokument WPI/DERWENT-Zusammenfassung 2001-613102 (JP-A-2001137279; SEKISUI) vom 22.05.2001 beschreibt ein Band für medizinische Behandlungen, welches eine Klebstoffschicht aufweist, die als antimikrobielle Komponente metallisches Silber enthält.

Die DE 197 56 790 A1 betrifft ein Polymer mit darin dispergierten Feststoffteilchen, die eine Teilchengröße von weniger als 20 nm aufweisen und im Wesentlichen isoliert darin dispergiert sind. Bei den Feststoffen kann es sich um Silber handeln.

Die antimikrobielle Wirksamkeit der nach dem Stand der Technik bekannten Materialien ist mit der so genannten Hemmhofmessung nachgewiesen worden. Die Remmhafmessung ist z.B. beschrieben in Raad I. et al., J. Infec. Dis. 173 (1996). Dabei wird das zu prüfende Material in ein Nährmedium, z.B. Agar, eingebettet. Wegen der Freisetzung antimikrobiell wirkender Metall-Ionen bildet sich um das Material ein Hemmhof. Die Ausbildung und die Größe eines solchen Hemmhofs ist nach dem Stand der Technik als Anzeichen für die antimikrobielle Wirksamkeit des Materials gewertet worden.

Die nach dem Stand der Technik bekannten Materialien haben den Nachteil, dass sie anfänglich eine zu hohe Konzentration an Silber-Ionen freisetzen. Materialien, welche einen Hemmhof zeigen, sind nach neueren Erkenntnissen nicht für den Einsatz im medizinischen Bereich geeignet. Solche Materialien geben Silber-Ionen in makroskopisch sichtbaren Mengen an das umgebende Gewebe ab. Derartige Mengen an Silber-Ionen sind jedoch zytotoxisch. So weit solche Materialien von den Zulassungsbehörden überhaupt zugelassen werden, erfolgt eine Zulassung inzwischen nur noch nach Arzneimittelrecht. Eine solche Zulassung ist äußerst kosten- und zeitaufwändig.

Insgesamt gesehen ist nach dem Stand der Technik bisher kein antimikrobieller Kleb- und Beschichtungsstoff bekannt, der im Hemmhoftest keinen Hemmhof zeigt und damit nicht zytotoxisch ist.

Aufgabe der Erfindung ist es, die Nachteile nach dem Stand der Technik zu beseitigen. Es soll insbesondere ein möglichst einfach und kostengünstig herstellbarer Kleb- und Beschichtungsstoff angegeben werden, der nicht zytotoxisch ist und dessen antimikrobielle Wirksamkeit möglichst lange anhält. Nach einem weiteren Ziel der Erfindung soll der Kleb- und Beschichtungsstoff möglichst universell einsetzbar sein und die Herstellung dünner Beschichtungen ermöglichen. Ferner soll ein einfaches und kostengünstiges Verfahren zur Herstellung des Kleb- und Beschichtungsstoffs angegeben werden.

Diese Aufgabe wird durch die Merkmale der Ansprüche 1, 23 und 24 gelöst. Zweckmäßige Ausgestaltungen ergeben sich aus den Merkmalen der Ansprüche 2 bis 22 und 25 bis 37.

Nach Maßgabe der Erfindung wird ein antimikrobieller Kleb- und Beschichtungsstoff mit den Merkmalen des Anspruchs 1 vorgeschlagen. Der vorgeschlagene Kleb- und Beschichtungsstoff weist eine besonders lang anhaltende antimikrobielle Wirkung auf. Er ist nicht zytotoxisch, d.h. im Hemmhoftest ist kein Hemmhof zu beobachten. Das wird darauf zurückgeführt, dass stetig eine geringe Rate an Silber-Ionen von den Silber-Partikeln freigegeben und an die Oberfläche des Kleb- und Beschichtungsstoffs diffundiert. Die geringe Freisetzungsrate wird nach derzeitigem Kenntnisstand durch den besonders geringen Gehalt an Silber-, Natrium- und Kalium-Ionen zurückgeführt, der insgesamt kleiner als 5 ppm ist. Die nach dem Stand der Technik bekannten Materialien enthalten überwiegend Silber-Partikel, die anfänglich eine hohe Rate an Silber-Ionen freisetzen. Eine hohe Freisetzungsrate an Silber-Ionen wird z. B. durch einen gestörten Gitteraufbau der Silber-Partikel oder durch einen hohen Gehalt an Ionen in den Silber-Partikeln erreicht. Gerade das ist nach dem Gegenstand der vorliegenden Erfindung nicht gegeben.

Unter dem Begriff Kleb- und Beschichtungsstoff ist vorliegend allgemein ein synthetisch hergestelltes Material auf organischer Basis zu verstehen. Das Material härtet im Allgemeinen nach der Verarbeitung aus. Es kann sich dabei um einen Anstrichstoff, um einen Lack, einen Klebstoff, insbesondere auch auf duro- oder thermoplastischer Basis handeln.

Nach einer ersten Ausgestaltung ist die mittlere Korngröße der Partikel kleiner als 100 nm, vorzugsweise im Bereich von 5 bis 50 nm. Solche Silber-Partikel eignen sich zur Herstellung nanodisperser antimikrabieller Kleb- und Beschichtungsstoffe.

Nach einer weiteren Ausgestaltung sind die Silber-Partikel aus Aggregaten von Primärpartikeln mit einer mittleren Korngröße zwischen 10 und 150 nm gebildet. Solche Silber-Partikel eignen sich zur Herstellung so genannter nanoporöser antimikrobieller Kleb- und Beschichtungsstoffe.

Nach einer vorteilhaften Ausgestaltung weisen die Primärpartikel eine mittlere Korngröße im Bereich von 80 bis 140 nm auf. Aus derartigen Primärpartikeln gebildete Silber-Partikel zeigen eine besonders gute antimikrobielle Wirksamkeit. Sie sind nicht zytotoxisch.

Nach einer vorteilhaften Ausgestaltung weisen die Aggregate eine mittlere Korngröße von 1 bis 20 µm, vorzugsweise 10 bis 20 µm, auf. Die Oberfläche der Aggregate beträgt zweckmäßigerweise 3 bis 6 m²/g. Sie können eine Porosität von bis zu 95% aufweisen. Zweckmäßigerweise liegt die Porosität zwischen 70 und 95%. Die vorgenannten Merkmale tragen zu einer gleichmäßigen und zytotoxisch unbedenklichen Abgabe von Silber-Ionen an der Oberfläche des Materials bei.

Zweckmäßigerweise ist der antimikrobielle Kleb- und Beschichtungsstoff aus mindestens einer flüssigen organischen Komponente hergestellt. Diese kann nach Art eines Lacks oder einer Anstrichfarbe durch das Verflüchtigen eines Lösungsmittels ausgehärtet werden. Es ist aber auch möglich, die organische Komponente zum Aushärten z.B. mittels UV-Licht, Wärme oder anderer physikalische Einflüsse zu polymerisieren. Der antimikrobielle Kleb- und Beschichtungsstoff kann auch durch Mischen der organischen Komponente mit mindestens einer weiteren flüssigen organischen Komponente hergestellt sein. Bei der weiteren organischen Komponente kann es sich z.B. um einen Härter handeln, der eine Polymerisation bewirkt. Die Silber-Partikel können unmittelbar der flüssigen organischen Komponente oder auch unmittelbar einem flüssigen Vorprodukt derselben zugesetzt sein. So gelingt es auf besonders einfache Weise, eine besonders homogene Dispersion der Silber- Partikel herzustellen. Es ist also insbesondere nicht erforderlich, mit hohem Zeit- und Kostenaufwand ein Zwischenprodukt herzustellen und dieses dann weiterzuverarbeiten.

Nach einer weiteren Ausgestaltung können 0,01 bis 5,0 Gew.% an Silber-Partikeln zugesetzt sein. Vorzugsweise sind 0,01 bis 2,0 Gew.% zugesetzt. Die erfindungsgemäß verwendeten Silber-Partikel sind bereits in einer geringen Konzentration langanhaltend antimikrobiell wirksam, ohne eine zytotoxische Wirkung hervorzurufen. Sie sind zweckmäßigerweise sphärisch, insbesondere kugelförmig, ausgebildet. Das erleichtert das Einmischen der Silber-Partikel in die flüssige organische Komponente. Es kann schnell eine homogene Dispersion hergestellt werden. Vorteilhafterweise sind die Aggregate vollständig mit der organischen Komponente infiltriert.

Die organische Komponente und/oder die weitere organische Komponente können als wesentlichen Bestandteil ein Acrylat oder ein Methacrylat enthalten. Sie können ferner als wesentlichen Bestandteil ein Epoxid, Urethan, Silikon oder Cyanacrylat enthalten.

In einer weiteren Ausgestaltung enthält der antimikrobielle Kleb- und Beschichtungsstoff als weiteren Zusatz Kationen mindestens eines der folgenden Metalle: Au, Pt, Pd, Ir, Sn, Cu, Sb, Zn. Der weitere Zusatz erhöht und/oder verlängert die antimikrobielle Wirksamkeit. Die Kationen sind zweckmäßigerweise gebunden in Ionentauschern, in Form eines Komplexes oder als Salz zugesetzt. Die Kationen können aus dem Kleb- und Beschichtungsstoff an dessen Oberfläche diffundieren und dort ihre antimikrobielle Wirksamkeit entfalten. In diesem Zusammenhang hat es sich auch als zweckmäßig erwiesen, als Salz das Salz einer, vorzugsweise polymeren, Carbonsäure zu verwenden.

Der Klebstoff kann ein Haftklebstoff sein. Die organische und/oder die weitere organische Komponente kann einen oder mehrere der folgenden Zusätze enthalten: Lösungsmittel, Füllstoff, Pigment, Bindemittel, Weichmacher, Trockenstoff, Fungizid. Der Beschichtungsstoff kann ein Lack, ein Anstrichstoff oder eine aushärtbare Dispersion sein.

Nach weiterer Maßgabe der Erfindung ist eine Verwendung des erfindungsgemäßen Kleb- und Beschichtungsstoffs zur Herstellung und/oder Beschichtung von Wundauflagen, Verbandsstoffen, Inkontinenzprodukte, z.B. Windeln, medizinischen Vorrichtungen, Verpackungsmitteln, zum Beschichten von Wänden von Gebäuden, Gehäusen und/oder Bauteilen technischer Vorrichtungen vorgesehen. Z.B. können die Gehäuseinnenwände von Klimaanlagen, die Wände von Operationssälen, Behälter zum Herstellen und Lagern von verderblichen Lebensmitteln, Verpackungsmittel für medizinische Einwegvorrichtungen, medizinische Einwegvorrichtungen, Verbandsmaterial, medizinische Instrumente usw. mit dem erfindungsgemäßen Material beschichtet oder daraus hergestellt sein.

Nach weiterer Maßgabe der Erfindung ist ein verfahren zur Herstellung des erfindungsgemäßen Kleb- und Beschichtungsstoffs mit folgenden Schritten vorgesehen:
Erzeugen eines Silberdampfs mittels Sputtern oder durch Verdampfen in einem Vakuumrezipienten,
Kondensieren des Silberdampfs, so dass Silber-Partikel mit Gehalt von weniger als 5 ppm an Silber-, Natrium- und Kalium-Ionen gebildet werden und
Versetzen einer flüssigen organischen Komponente mit den Silber-Partikeln.

Das vorgeschlagene Verfahren ist einfach und kostengünstig durchführbar.

Es kann damit ein antimikrobieller Kleb- und Beschichtungsstoff bereitgestellt werden, dessen antimikrobielle Wirkung besonders langanhaltend ist und der gleichzeitig keine zytotoxische Wirkung auf in Kontakt damit befindliches Gewebe hat. Mit dem Verfahren können feste Kleb- und Beschichtungsstoffe und teilweise aushärtende Haftklebstoffe hergestellt werden. Das Verfahren eignet sich auch allgemein zur Herstellung von Kunststoffen, vorzugsweise von aus mindestens einer flüssigen Komponente hergestellten Kunststoffen. Aus solchen Kunststoffen können z.B. medizinische Vorrichtungen, wie Katheter und dgl. hergestellt werden.

Zweckmäßigerweise wird die organische Komponente nach dem Versetzen mit Silber-Partikeln zumindest teilweise ausgehärtet.

Bei einer ersten Verfahrensvariante wird der Silberdampf in der Gasphase kondensiert, so dass aus Primärpartikeln mit einer mittleren Korngröße von 10 bis 150 nm bestehende Aggregate gebildet werden, die eine mittlere Korngröße von 1 bis 20 µm aufweisen.

Bei dieser Verfahrensvariante bilden sich aus der Dampfphase zunächst die Primärpartikel. Die gebildeten Primärpartikel weisen eine extrem hohe Oberflächenenergie auf. Bei einer Aneinanderlagerung der Primärpartikel kommt es bereits bei der gegebenen Aktivierungsenergie zu Diffusionsprozessen, welche die Ausbildung von Sinterhälsen zur Folge haben. Es bilden sich Aggregate, bei denen die Primärpartikel durch Sinterhälse miteinander verbunden sind. Auf diese Weise können hochporöse Aggregate gebildet werden.

Nach einer weiteren Verfahrensvariante wird der Silberdampf an der Oberfläche einer Flüssigkeit kondensiert, so dass die Silber-Partikel eine mittlere Korngröße im Bereich von 10 bis 100 nm aufweisen. Bei dieser Verfahrensvariante wird die Bildung von Agglomeraten verhindert, indem durch eine Kondensation an einer Flüssigkeitsoberfläche die zur Bildung von Sinterhälsen erforderliche Aktivierungsenergie den Silber-Partikeln entzogen wird. Auf diese Weise können nanodisperse antimikrobielle Kleb- und Beschichtungsstoffe hergestellt werden.

Bei der Flüssigkeit kann es sich um ein Vorprodukt der organischen Komponente oder die organische Komponente handeln. Das vorgeschlagene Verfahren ist besonders einfach durchführbar. Es kann in einer einzigen Vorrichtung eine homogene Dispersion der Silber-Partikel in der organischen Komponente oder deren Vorprodukt hergestellt werden. Der Kleb- und Beschichtungsstoff kann durch Aushärten der organischen Komponente, z.B. durch Hinzufügen einer weiteren organischen Komponente, weiterverarbeitet werden.

Wegen weiterer vorteilhafter Ausgestaltungen wird auf die bereits beim antimikrobiellen Kleb- und Beschichtungsstoff beschriebenen Merkmale verwiesen, welche sinngemäß auch im Zusammenhang mit dem Verfahren anwendbar sind.

Nachfolgend werden anhand der Zeichnung Ausführungsbeispiele der Erfindung erläutert. Es zeigen:
- Fig. 1: Silber-Partikel dispergiert in Polytetrahydrofuran,
- Fig.2: Silber-Partikel dispergiert in Diethylhexylphthalat,
- Fig.3: Silber-Partikel dispergiert in Dimethylsiloxan,
- Fig.4: den Nachweis der antimikrobiellen Wirkung eines mit Silber-Partikel versetzten Epoxidharz-Klebstoffs,
- Fig.5: eine rasterelektronenmikroskopische Aufnahme eines Silberaggregats und
- Fig.6: einen Hemmhoftest eines erfindungsgemäßen Beschichtungsstoffs.

### Herstellung der metallischen Silber-Partikel:

Silber (99,95 Silberdraht der Firma Heraeus) wird durch Magnetron-Sputtern in einem Vakuumrezipienten bei einer Temperatur von 20°C und einem Druck von 0,01 bis 0,1 mbar in Argonatmosphäre verdampft. Die Verdampfung kann im Vakuumrezipienten auch thermisch, z. B. in einem Schmelztiegel, erfolgen. Der Silberdampf wird anschließend auf der Oberfläche einer Flüssigkeit abgeschieden oder zusammen mit der verdampften Flüssigkeit auf einer gekühlten Fläche kondensiert. Bei der Flüssigkeit kann es sich z. B. um Silikonöl, Polytetrahydrofuran, Diethylhexylphthalat oder Dimethylsiloxan handeln. Dabei wird die Flüssigkeit kontinuierlich durchmischt, so dass die abgeschiedenen bzw. kondensierten metallischen Silber-Partikel in der Flüssigkeit homogen dispergiert werden. Als Flüssigkeit wird vorzugsweise die organische Komponente oder ein Vorprodukt der organischen Komponente des herzustellenden Kleb- und Beschichtungsstoffs verwendet.

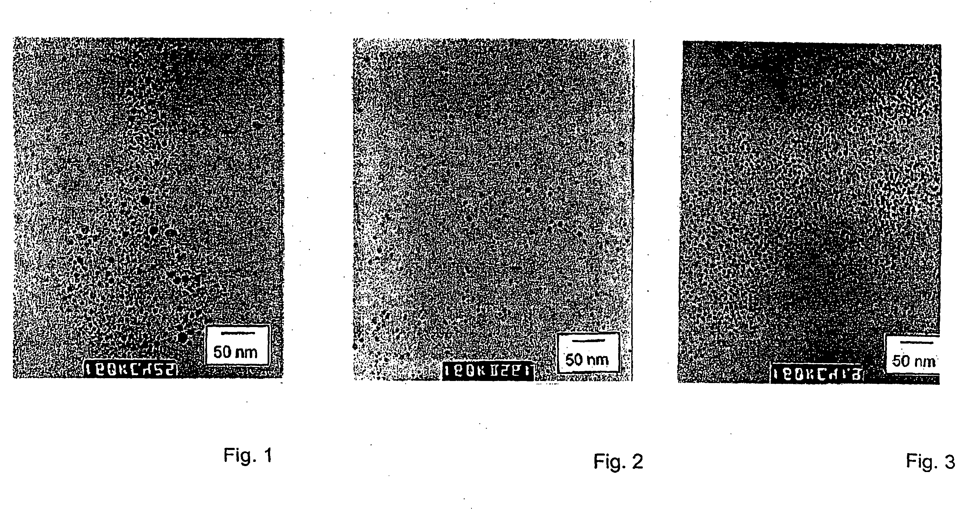

Fig.1 zeigt nach dem vorgenannten Verfahren dispergierte sphärische Silber-Partikel in Polytetrahydrofuran. Es handelt sich dabei um ein Vorprodukt eines Epoxidharzes. Aus Fig.1 ist ersichtlich, dass die Silber-Partikel eine mittlere Korngröße von weniger als 10 nm aufweisen.

Fig.2 zeigt sphärische Silber-Partikel, welche nach dem vorgenannten Verfahren in Diethylhexylphthalat dispergiert worden sind. Es handelt sich dabei um eine Komponente für einen Anstrich aus Polyvinylchlorid (PVC). Auch hier liegt eine homogene Dispersion der Silber-Partikel in der Komponente vor. Die Silber-Partikel weisen eine mittlere Korngröße von weniger als 10 nm auf.

Fig.3 zeigt eine Dispersion von kugelförmigen Silber-Partikeln in einem Dimethylsiloxan. Es handelt sich dabei um eine Komponente eines Silikonklebers. Die Silber-Partikel weisen hier wiederum eine mittlere Korngröße von weniger als 10 nm auf.

Die vorbeschriebenen Dispersionen können anschließend, z.B. durch Zugabe einer weiteren flüssigen Komponente, ausgehärtet werden. Sie können auch mit funktionellen Gruppen, z.B. Acrylat- oder Methacrylatgruppen, versetzt oder z.B. im Falle des Polytetra-hydrofurans, copolymerisiert werden.

Nachfolgend werden Beispiele zur Herstellung der antimikrobiellen Kleb- und Beschichtungsstoffe beschrieben.

### Beispiel 1

### Präparation von bakteriziden Silikonmaterialien:

Eine Komponente, z.B. ein Binder, eines additionsvernetzenden Zweikomponenten-Silikonkautschuks auf Basis von Polydimethylphenylsiloxan (RTV-S691; Wacker) wird mit nach dem oben beschriebenen Verfahren hergestellten Silber-Partikeln dotiert. Die Komponente wird anschließend im Verhältnis 9:1 mit einer weiteren Komponente, z.B. einem Härter, gemischt. Die Aushärtung erfolgt bei T=25°C. Die resultierende Konzentration an Silber im ausgehärteten Material beträgt dabei 0,01 bis 5 Gew.%, vorzugsweise 0,05 - 1 Gew.%, Silber. Das aushärtbare Material lässt sich unmittelbar als Klebstoff, Lack oder nach Zugabe geeigneter Pigmente bzw. Farbstoffe auch als Druckfarbe verwenden.

### Beispiel 2

### Präparation von bakteriziden Epoxidharzklebstoffen:

Ein Härter (HV 998, Ciba) eines pastösen Zweikomponentenklebstoff auf Epoxidharzbasis (Araldit AV138M, Ciba) wird nach dem oben beschriebenen Verfahren mit Silber-Nanopartikeln dotiert. Der dotierte Härter wird in das Epoxidharz eingerührt und bei Raumtemperatur ausgehärtet. Die Konzentration an Silber beträgt dabei 0,01 bis 5 Gew.%, vorzugsweise 0,1 bis 1 Gew.%, Silber im ausgehärteten Zweikomponentenklebstoff. Das aushärtbare Material lässt sich unmittelbar als Klebstoff, Lack oder nach Zugabe geeigneter Pigmente bzw. Farbstoffe z.B. als Druckfarbe verwenden.

### Beispiel 3

### Präparation bakterizider und bakteriostatischer Epoxidharzklebstoffe:

Es werden Klebstoffe auf Basis kationisch härtender cycloaliphatischer Epoxidharze präpariert. Das Epoxidharz ERL 4221 (Union Carbide) wird mit Polytetrahydrofuran (PTHF) der Molmasse 1000 (PTHF 1000, BASF) copolymerisiert. PTHF dient zur Flexibilisierung bzw. als Weichmacher. Dabei enthält das PTHF 5 Gew.% über das oben beschriebene Verfahren eingebrachtes Silber (Bezeichnung: PTHF-VERL-Ag). Hieraus resultiert ein Silbergehalt der erfindungsgemäßen Proben von 1 Gew.%. Bei den Vergleichsbeispielen ist kein Silber enthalten. Die thermische kationische Härtung erfolgt unter Zusatz eines Initiators. Als Initiator kann z.B. das Iodoniumsalz Rhodorsil 2074 (Rhodia) mit Ascorbinsäure-6-hexadecanat (ASHD) als Beschleuniger und zum anderen mit α,α-Dimethyl-benzylpyridinium hexafluoroantimonat (S. Nakano, T. Endo, J. Polym. Sci.: Part A, 34 (1996) 475) verwendet werden. Die Härtung erfolgt mit folgendem Temperaturprogramm: 90 min 80°C, 60 min 100°C und 60 min 120°C.

Die Zusammensetzung der einzelnen Mischungen in Gew.% und die mikrobiologische Wirkung auf Staphylokkokus epidermidis ist in der folgenden Tabelle zusammengestellt.

| Harz | Flexibilisator | Initiator | Art des Beispiels | mikrobiologische Wirkung |
|---|---|---|---|---|
| 79% ERL 4221 | 20% PTHF | 1% α,α-Dimethylbenzylpyridinium hexafluoroantimonat | Vergleich | keine |
| 78% ERL 4221 | 20% PTHF | 1% Rhodorsil 2074, 1% ASHD | Vergleich | keine; Fig. 4, Probe A |
| 79% ERL 4221 | 20% PTHF-VERL-Ag | 1% α,α-Dimethylbenzylpyridinium hexafluoroantimonat | erfindungsgemäß | bakteriostatisch; Fig. 4, Probe B |
| 78% ERL 4221 | 20% PTHF-VERL-Ag | 1% Rhodorsil 2074, 1% ASHD | erfindungsgemäß | bakterizid; Fig. 4, Probe C |

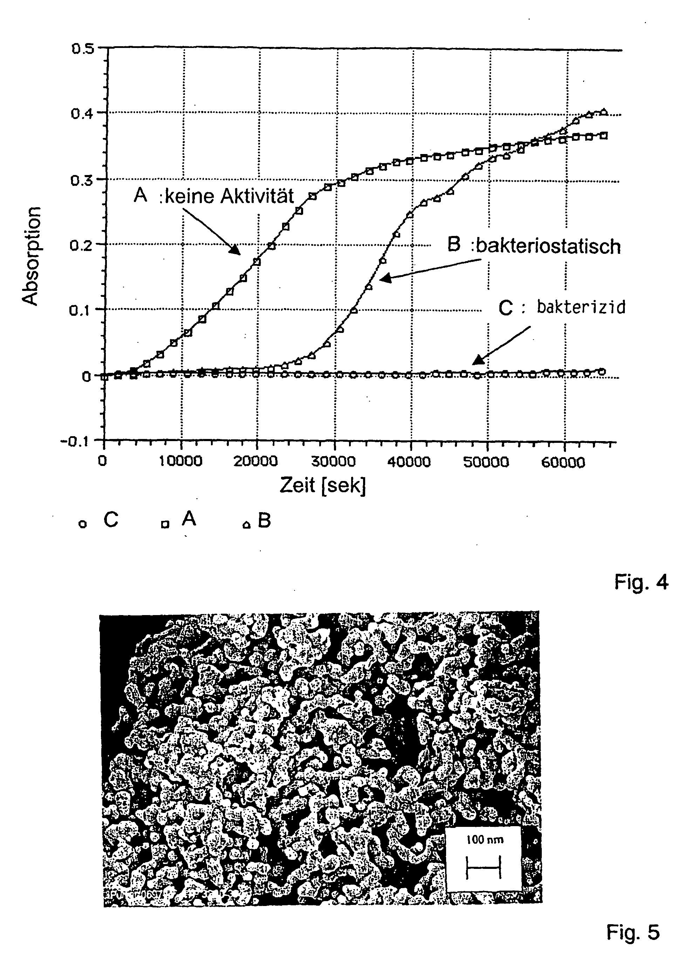

Die in den Fig. 4 gezeigten Ergebnisse sind nach dem aus der DE 197 51 581 A1 bekannten Verfahren ermittelt worden. Dieses Verfahren ist ferner beschrieben in Bechert, Thorsten et al., Nature Medicine, Vol. 6, No. 8 (09/2000). Der Offenbarungsgehalt der beiden vorgenannten Dokumente wird hiermit einbezogen.

Es werden zunächst jeweils 8 Parallelproben derselben Charge des Epoxidharzklebstoffs angefertigt. Die Proben sind üblicherweise zylinderförmig ausgebildet. Sie weisen eine Länge von etwa 1 cm und einen Durchmesser von 2 bis 5 mm auf. Anschließend wird in jede Vertiefung der Mikrotiterplatte 200 µl der Bakterien-enthaltenden Lösung gefüllt. Die Proben werden bei 37°C für eine Stunde inkubiert. Die Proben werden dann entnommen und dreimal mit physiologischem Puffern gewaschen. Dann werden die Proben in die Vertiefungen einer Mikrotiterplatte gelegt, welche mit einem Minimalmedium gefüllt sind. Pro Vertiefung werden 200 µl an Minimalmedium eingefüllt. Die Proben werden für 24 Stunden bei 37°C inkubiert. Anschließend werden die Proben entnommen und verworfen. Zu jeder Vertiefung der Mikrotiterplatte werden 50 µl eines Vollmediums (Trypcasesoja) zugegeben. Anschließend wird die Trübung bzw. Absorption der Lösung im Abstand von 30 Minuten über einen Zeitraum von 48 Stunden gemessen. Die Lösung wird dabei auf einer Temperatur von 37°C gehalten. Die Trübungsmessung erfolgt mit Licht einer Wellenlänge von 578 nm mittels eines geeigneten Lesegeräts. Eine Trübung zeigt an, dass Bakterien von der Oberfläche der Probe in die Umgebung abgegeben worden sind.

Die in Fig. 4 dargestellten Ergebnisse zeigen, dass durch geeignete Wahl des Initiators die bakterizide Wirkung der Klebstoffe gesteuert werden kann. Das aushärtbare Material lässt sich unmittelbar als Klebstoff, Lack oder nach Zugabe geeigneter Pigmente bzw. Farbstoffe z.B. als Druckfarbe verwenden.

Fig. 5 zeigt eine rasterelektronenmikroskopische Aufnahme des erfindungsgemäßen Silberaggregats. Das Silberaggregat besteht im Wesentlichen aus kugeligen Primärpartikeln mit einer mittleren Korngröße von etwa 20 nm. Die Primärpartikel sind im Wesentlichen über Sinterhälse miteinander verbunden. Sie bilden ein hochporöses Gerüst. Das hier gezeigte Silberaggregat hat eine Größe von etwa 10 µm. Solche Silber-Aggregate können anstelle der in den vorhergehenden Beispielen beschriebenen feinen Silber-Partikel ebenfalls dem Kleb- und Beschichtungsstoff zugesetzt sein. Es werden damit vergleichbare Ergebnisse hinsichtlich der antimikrobiellen Wirkung sowie im Hemmhoftest erzielt.

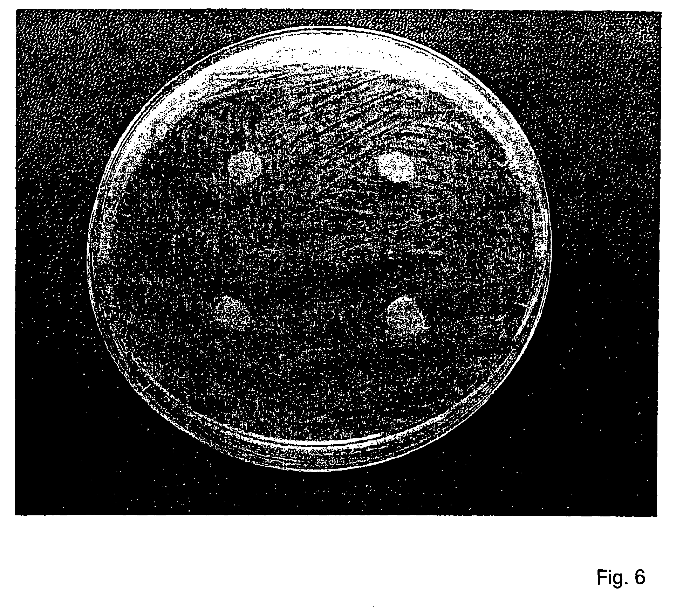

Fig. 6 zeigt einen Hemmhoftest eines erfindungsgemäßen Kleb- und Beschichtungsstoffs. Es handelt sich dabei um einen Polyurythan-Lack, dem 0,1 Gew.% Silber-Partikel zugesetzt sind. Bei den Silber-Partikeln handelt es sich um Agglomerate, wie sie in Fig. 5 gezeigt sind. Die Proben sind in TSB-Agar als Nährmedium eingesetzt, dem als Testorganismus Staphylokkokus epidermidis zugefügt ist. Die Proben sind 48 Stunden bebrütet worden. Wie aus Fig. 6 ersichtlich ist, zeigen die Proben keinen Hemmhof. Sie werden insoweit nicht als zytotoxisch wirkend angesehen.

## Patentansprüche

1. Antimikrobieller Kleb- und Beschichtungsstoff, der als antimikrobielle Komponente metallische Silber-Partikel mit einem Gehalt von weniger als 5 ppm an Silber-, Natrium- und Kalium-Ionen enthält, hergestellt nach einem Verfahren mit den folgenden Schritten:
Erzeugen eines Silberdampfs mittels Sputtern oder durch verdampfen in einem Vakuumrezipienten,
Kondensieren des Silberdampfs, so dass Silber-Partikel mit einem Gehalt von weniger als 5 ppm an Silber-, Natrium und Kalium-Ionen Gehalt gebildet werden und
Versetzen einer flüssigen organischen Komponente mit den Silber-Partikeln.

2. Antimikrobieller Kleb- und Beschichtungsstoff nach Anspruch 1, wobei im Verfahren die organische Komponente nach dem Versetzten mit Silber-Partikeln zumindest teilweise ausgehärtet wird.

3. Antimikrobieller Kleb- und Beschichtungsstoff nach Anspruch 1 oder 2, wobei die mittlere Korngröße der Silber-Partikel kleiner als 100 nm, vorzugsweise im Bereich von 5 bis 50 nm, ist.

4. Antimikrobieller Kleb- und Beschichtungsstoff nach einem der Ansprüche 1 oder 2, wobei die Silber-Partikel aus Aggregaten von Primärpartikeln mit einer mittleren Korngröße zwischen 10 und 150 nm gebildet sind.

5. Antimikrobieller Kleb- und Beschichtungsstoff nach Anspruch 4, wobei die Primärpartikel eine mittlere Korngrö-ße im Bereich von 80 bis 140 nm aufweisen.

6. Antimikrobieller Kleb- und Beschichtungsstoff nach Anspruch 4 oder 5, wobei die Aggregate eine mittlere Korngröße von 1 bis 20 µm, vorzugsweise 10 bis 20 µm, aufweisen.

7. Antimikrobieller Kleb- und Beschichtungsstoff nach einem der Ansprüche 4 bis 6, wobei die Aggregate eine Porosität von bis zu 95% aufweisen.

8. Antimikrobieller Kleb- und Beschichtungsstoff nach einem der Ansprüche 4 bis 7, wobei die Aggregate eine Oberfläche von 3 bis 6 m² pro Gramm aufweisen.

9. Antimikrobieller Kleb- und Beschichtungsstoff nach einem der vorhergehenden Ansprüche, wobei das Silber einen im Wesentlichen ungestörten Gitteraufbau aufweist.

10. Antimikrobieller Kleb- und Beschichtungsstoff nach einem der vorhergehenden Ansprüche, wobei er aus mindestens einer flüssigen organischen Komponente hergestellt ist.

11. Antimikrobieller Kleb- und Beschichtungsstoff nach einem der vorhergehenden Ansprüche, wobei er durch Mischen der organischen Komponente mit mindestens einer weiteren organischen Komponente hergestellt ist.

12. Antimikrobieller Kleb- und Beschichtungsstoff nach einem der vorhergehenden Ansprüche, wobei die Silber-Partikel unmittelbar der flüssigen organischen Komponente oder unmittelbar einem flüssigen Vorprodukt derselben zugesetzt sind.

13. Antimikrobieller Kleb- und Beschichtungsstoff nach einem der vorhergehenden Ansprüche, wobei 0,01 bis 5,0 Gew.% an Silber-Partikeln zugesetzt sind.

14. Antimikrobieller Kleb- und Beschichtungsstoff nach einem der vorhergehenden Ansprüche, wobei die Aggregate mit der organischen Komponente vollständig infiltriert sind.

15. Antimikrobieller Kleb- und Beschichtungsstoff nach einem der vorhergehenden Ansprüche, wobei die organische Komponente und/oder die weitere organische Komponente als wesentlichen Bestandteil ein Acrylat- oder ein Methacrylat enthält.

16. Antimikrobieller Kleb- und Beschichtungsstoff nach einem der vorhergehenden Ansprüche, wobei die organische Komponente und/oder die weitere organische Komponente als wesentlichen Bestandteil ein Epoxid, Urethan, Silikon oder Cyanacrylat enthält.

17. Antimikrobieller Kleb- und Beschichtungsstoff nach einem der vorhergehenden Ansprüche, wobei als weiterer Zusatz Kationen mindestens eines der folgenden Metalle enthalten sind: Au, Pt, Pd, Ir, Sn, Cu, Sb, Zn.

18. Antimikrobieller Kleb- und Beschichtungsstoff nach Anspruch 17, wobei die Kationen gebunden in Ionentauschern, in Form eines Komplexes oder als Salz zugesetzt sind.

19. Antimikrobieller Kleb- und Beschichtungsstoff nach vorhergehenden Anspruch 18, wobei das Salz ein Salz einer, vorzugsweise polymeren, Carbonsäure ist.

20. Antimikrobieller Kleb- und Beschichtungsstoff nach einem der vorhergehenden Ansprüche, wobei der Kleb- und Beschichtungsstoff ein Klebstoff, insbesondere ein Haftklebstoff, ist.

21. Antimikrobieller Kleb- und Beschichtungsstoff nach einem der vorhergehenden Ansprüche, wobei die organische und/oder die weitere organische Komponente einen oder mehrere der folgenden Zusätze enthält: Lösungsmittel, Füllstoff, Pigment, Bindemittel, Weichmacher, Trockenstoff, Fungizid.

22. Antimikrobieller Kleb- und Beschichtungsstoff nach einem der vorhergehenden Ansprüche, wobei der Kleb- und Beschichtungsstoff ein Lack, ein Anstrichstoff oder eine aushärtbare Dispersion ist.

23. Verwendung des Kleb- und Beschichtungsstoffs nach einem der vorhergehenden Ansprüche zur Herstellung und/oder Beschichtung von Wundauflagen, Verbandsstoffen, Inkontinenzprodukte, medizinischen Vorrichtungen, Verpackungsmitteln, zum Beschichten von Wänden von Gebäuden, Gehäusen und/oder Bauteilen technischer Vorrichtungen.

24. Verfahren zur Herstellung eines Kleb- und Beschichtungsstoffs nach einem der Ansprüche 1 bis 22 mit folgenden Schritten:
Erzeugen eines Silberdampfs mittels Sputtern oder durch Verdampfen in einem Vakuumrezipienten,
Kondensieren des Silberdampfs, so dass Silber-Partikel mit einem Gehalt von weniger als 5 ppm an Silber-, Natrium und Kalium-Ionen Gehalt gebildet werden und
Versetzen einer flüssigen organischen Komponente mit den Silber-Partikeln.

25. Verfahren nach Anspruch 24, wobei die organische Komponente nach dem Versetzten mit Silber-Partikeln zumindest teilweise ausgehärtet wird.

26. Verfahren nach Anspruch 24 oder 25, wobei der Silberdampf in der Gasphase kondensiert wird, so dass aus Primärpartikeln mit einer mittleren Korngröße von 10 bis 150 nm bestehende Aggregate gebildet werden, die eine mittlere Korngröße von 1 bis 20 µm aufweisen.

27. Verfahren nach Anspruch 24 oder 25, wobei der Silberdampf an der Oberfläche einer Flüssigkeit kondensiert wird, so dass die Silber-Partikel eine mittlere Korngröße im Bereich von 10 bis loo nm aufweisen.

28. Verfahren nach einem der Ansprüche 24 bis 27, wobei als Flüssigkeit die organische Komponente oder ein Vorprodukt derselben verwendet wird.

29. Verfahren nach einem der Ansprüche 24 bis 28, wobei die Silberpartikel anstelle der organischen Komponente einem Vorprodukt derselben zugesetzt und nachfolgend die organische Komponente hergestellt wird.

30. Verfahren nach einem der Ansprüche 24 bis 29, wobei 0,01 bis 5,0 Gew.% an Silber-Partikeln der organischen Komponente zugesetzt werden.

31. Verfahren nach einem der Ansprüche 24 bis 30, wobei die organische Komponente mit mindestens einer weiteren flüssigen organischen Komponente gemischt wird.

32. Verfahren nach einem der Ansprüche 24 bis 31, wobei die organische Komponente und/oder die weitere organische Komponente als wesentlichen Bestandteil ein Acrylat- oder ein Methacrylat enthält.

33. Verfahren nach einem der Ansprüche 24 bis 32, wobei die organische Komponente und/oder die weitere organische Komponente als wesentlichen Bestandteil ein Epoxid, Urethan, Silikon oder Cyanacrylat enthält.

34. Verfahren nach einem der Ansprüche 24 bis 33, wobei der organischen Komponente und/oder der weiteren organischen Komponente Kationen mindestens eines der folgenden Metalle zugesetzt wird/werden: Au, Pt, Pd, Ir, Sn, Cu, Sb, Zn.

35. Verfahren nach Anspruch 34, wobei die Kationen gebunden in Ionentauschern, in Form eines Komplexes oder als Salz zumindest der ersten organischen Komponente zugesetzt werden.

36. Verfahren nach Anspruch 35, wobei das Salz ein Salz einer, vorzugsweise polymeren, Carbonsäure ist.

37. Verfahren nach einem der Ansprüche 24 bis 36, wobei der organischen Komponente und/oder der weiteren organischen Komponente einer oder mehrere der folgenden Zusätze zugesetzt wird/werden: Lösungsmittel, Füllstoff, Pigment, Bindemittel, Weichmacher, Trockenstoff, Fungizid.

## Claims

1. Antimicrobial adhesive and coating material which contains as antimicrobial component metallic silver particles with a content of less than 5 ppm of silver, sodium and potassium ions, produced with a method comprising of the following steps:
Generation of a silver vapor via sputtering or by vaporization in a vacuum receiver,
Condensation of the silver vapor so that silver particles with a content of less than 5 ppm of silver, sodium and potassium ions are created and
Mixing silver particles into a liquid organic component.

2. Antimicrobial adhesive and coating material as defined in claim 1, wherein, with the method, the organic component is at least partially hardened after being mixed with silver particles.

3. Antimicrobial adhesive and coating material as defined in claim 1 or 2, wherein the mean grain size of the silver particles is less than 100 nm, preferably in the range of 5 to 50 nm.

4. Antimicrobial adhesive and coating material as defined in one of the claims 1 or 2, wherein the silver particles are created from aggregations of primary particles with a mean grain size of between 10 and 150 nm.

5. Antimicrobial adhesive and coating material as defined in claim 4, wherein the primary particles have a mean grain size in the range of 80 to 140 nm.

6. Antimicrobial adhesive and coating material as defined in claim 4 or 5, wherein the aggregates have a mean grain size of 1 to 20 µm, preferably 10 to 20 µm.

7. Antimicrobial adhesive and coating material as defined in one of the claims 4 to 6, wherein the aggregates have a porosity of up to 95%.

8. Antimicrobial adhesive and coating material as defined in one of the claims 4 to 7, wherein the aggregates have a surface of 3 to 6 m² per gram.

9. Antimicrobial adhesive and coating material as defined in one of the preceding claims, wherein the silver has a essentially undisturbed lattice structure.

10. Antimicrobial adhesive and coating material as defined in one of the preceding claims, wherein it is made from at least one liquid, organic component.

11. Antimicrobial adhesive and coating material as defined in one of the preceding claims, wherein it is made by mixing the organic component with at least one further organic component.

12. Antimicrobial adhesive and coating material as defined in one of the preceding claims, wherein the silver particles are directly added to the liquid organic component or directly to a liquid pre-product of same.

13. Antimicrobial adhesive and coating material as defined in one of the preceding claims, wherein 0.01 to 5.0 percent in weight of silver particles are added.

14. Antimicrobial adhesive and coating material as defined in one of the preceding claims, wherein the aggregates are completely infiltrated with the organic component.

15. Antimicrobial adhesive and coating material as defined in one of the preceding claims, wherein the organic component and/or the further organic component contains as essential ingredient an acrylate or a methacrylate.

16. Antimicrobial adhesive and coating material as defined in one of the preceding claims, wherein the organic component and/or the further organic component contains as essential ingredient an epoxy, urethane, silicone or cyanoacrylate.

17. Antimicrobial adhesive and coating material as defined in one of the preceding claims, wherein as a further additive cations are contained of at least one of the following metals: Au, Pt, Pd, Ir, Sn, Cu, Sb, Zn.

18. Antimicrobial adhesive and coating material as defined in claim 17, wherein the cations are added being bonded in ion exchangers, in the form of a complex or as salt.

19. Antimicrobial adhesive and coating material as defined in claim 18, wherein the salt is a salt of a, preferably polymer, carboxylic acid.

20. Antimicrobial adhesive and coating material as defined in one of the preceding claims, wherein the adhesive and coating material is an adhesive, in particular a pressure-sensitive adhesive.

21. Antimicrobial adhesive and coating material as defined in one of the preceding claims, wherein the organic and/or the further organic component contains one or more of the following additives: solvent, filler, pigment, binding agent, plasticizer, drying accelerator, fungicide.

22. Antimicrobial adhesive and coating material as defined in one of the preceding claims, wherein the adhesive and coating material is a lacquer, a paint substance or a hardenable dispersion.

23. Use of the adhesive and coating material as defined in one of the preceding claims, for the production and/or coating of wound coverings, bandaging materials, incontinence products, medical devices, packaging agents, for the coating of walls of buildings, cabinets and/or components of technical devices.

24. Method for the making of an adhesive and coating material as defined in one of the claims 1 to 22, consisting of the following steps:
Generation of a silver vapor via sputtering or by vaporization in a vacuum receiver,
Condensation of the silver vapor so that silver particles with a content of less than 5 ppm of silver, sodium and potassium ions are created and
Addition of silver particles to a liquid organic component.

25. Method as defined in claim 24, wherein the organic component is at least partially hardened after silver particles are added.

26. Method as defined in claim 24 or 25, wherein the silver vapor is condensed during the vapor phase so that aggregates consisting of primary particles with a mean grain size of 10 to 150 nm are created which have a mean grain size of 1 to 20 µm.

27. Method as defined in claim 24 or 25, wherein the silver vapor is condensed on the surface of a liquid so that the silver particles have a mean grain size in the range from 10 to 100 nm.

28. Method as defined in one of the claims 24 to 27, wherein the organic component or a pre-product of same is used as the liquid.

29. Method as defined in one of the claims 24 to 28, wherein the silver particles are added to a pre-product of same instead of the organic component and then the organic component is made.

30. Method as defined in one of the claims 24 to 29, wherein 0.01 to 5.0 percent in weight of silver particles is added to the organic component.

31. Method as defined in one of the claims 24 to 30, wherein the organic component is mixed with at least one further liquid organic component.

32. Method as defined in one of the claims 24 to 31, wherein the organic component and/or the further organic component contains as essential ingredient an acrylate or a methacrylate.

33. Method as defined in one of the claims 24 to 32, wherein the organic component and/or the further organic component contains as essential ingredient an epoxy, urethane, silicone or cyanoacrylate.

34. Method as defined in one of the claims 24 to 33, wherein cations of at least one of the following metals is/are added to the organic component and/or the further organic component: Au, Pt, Pd, Ir, Sn, Cu, Sb, Zn.

35. Method as defined in claim 34, wherein the cations are added being bonded in ion exchangers, in the form of a complex or as salt to at least the first organic component.

36. Method as defined in claim 35, wherein the salt is a salt of a, preferably polymer, carboxylic acid.

37. Method as defined in one of the claims 24 to 36, wherein one or more of the following additives is/are added to the organic component and/or the further organic component: solvent, filler, pigment, binding agent, plasticizer, drying accelerator, fungicide.

## Revendications

1. Matériau d'adhésion et de revêtement antimicrobien, qui, au titre de composante antimicrobien, contient des particules métalliques d'argent dont la teneur en ions argent, sodium et potassium est inférieure à 5 ppm, fabriqué selon un procédé comprenant les étapes suivantes :
- produire une vapeur d'argent par pulvérisation ou évaporation dans un récipient sous vide,
- condenser de la vapeur d'argent, de telle sorte que des particules d'argent dont la teneur en ions argent, sodium et potassium est inférieure à 5 ppm, sont formées, et
- mélanger une composante organique liquide avec les particules d'argent.

2. Matériau d'adhésion et de revêtement antimicrobien selon la revendication 1, la composante organique étant dans le procédé au moins partiellement durcie après le mélange avec les particules d'argent.

3. Matériau d'adhésion et de revêtement antimicrobien selon la revendication 1 ou 2, dans lequel la granulométrie moyenne des particules d'argent est inférieure à 100 nm, de préférence est comprise dans la plage de 5 à 50 nm.

4. Matériau d'adhésion et de revêtement antimicrobien selon la revendication 1 ou 2, dans lequel les particules d'argent se composent d'agrégats de particules primaires dont la granulométrie moyenne est comprise entre 10 et 150 nm.

5. Matériau d'adhésion et de revêtement antimicrobien selon la revendication 4, dans lequel les particules primaires ont une granulométrie moyenne dans la plage de 80 à 140 nm.

6. Matériau d'adhésion et de revêtement antimicrobien selon la revendication 4 ou 5, dans lequel les agrégats ont une granulométrie moyenne comprise entre 1 et 20 µm, de préférence entre 10 et 20 µm.

7. Matériau d'adhésion et de revêtement antimicrobien selon l'une des revendications 4 à 6, dans lequel les agrégats présentent une porosité allant jusqu'à 95 %.

8. Matériau d'adhésion et de revêtement antimicrobien selon l'une des revendications 4 à 7, dans lequel les agrégats présentent une surface de 3 à 6 m² par gramme.

9. Matériau d'adhésion et de revêtement antimicrobien selon l'une quelconque des revendications précédentes, dans lequel l'argent présente une structure de réseau sensiblement non dérangée ou perturbée.

10. Matériau d'adhésion et de revêtement antimicrobien selon l'une quelconque des revendications précédentes, fabriqué à partir d'au moins une composante organique liquide.

11. Matériau d'adhésion et de revêtement antimicrobien selon l'une quelconque des revendications précédentes, fabriqué en mélangeant la composante organique avec au moins une composante organique supplémentaire.

12. Matériau d'adhésion et de revêtement antimicrobien selon l'une quelconque des revendications précédentes, dans lequel les particules d'argent sont directement ajoutées à la composante organique liquide ou directement à un pré-produit liquide de celle-ci.

13. Matériau d'adhésion et de revêtement antimicrobien selon l'une quelconque des revendications précédentes, dans lequel sont ajoutées 0,01 à 5 % en poids de particules d'argent.

14. Matériau d'adhésion et de revêtement antimicrobien selon l'une quelconque des revendications précédentes, dans lequel les agrégats sont totalement infiltrés par la composante organique.

15. Matériau d'adhésion et de revêtement antimicrobien selon l'une quelconque des revendications précédentes, dans lequel la composante organique et/ou la composante organique supplémentaire comprend et/ou comprennent un acrylate ou un méthacrylate en tant que constituant essentiel.

16. Matériau d'adhésion et de revêtement antimicrobien selon l'une quelconque des revendications précédentes, dans lequel la composante organique et/ou la composante organique supplémentaire comprend et/ou comprennent une résine époxy, un uréthane, une silicone ou un cyanoacrylate en tant que constituant essentiel.

17. Matériau d'adhésion et de revêtement antimicrobien selon l'une quelconque des revendications précédentes, comprenant en tant que produit d'addition supplémentaire des cations provenant au moins de l'un des métaux suivants : Au, Pt, Pd, Ir, Sn, Cu, Sb, Zn.

18. Matériau d'adhésion et de revêtement antimicrobien selon la revendication 17, dans lequel les cations liés dans des dispositifs échangeurs d'ions, sont ajoutés sous la forme d'un complexe ou d'un sel.

19. Matériau d'adhésion et de revêtement antimicrobien selon la revendication 18, le sel étant un sel d'un acide carboxylique, de préférence polymérique.

20. Matériau d'adhésion et de revêtement antimicrobien selon l'une quelconque des revendications précédentes, le matériau d'adhésion et de revêtement antimicrobien étant une colle, en particulier une colle autoadhésive.

21. Matériau d'adhésion et de revêtement antimicrobien selon l'une quelconque des revendications précédentes, dans lequel la composante organique et/ou la composante organique supplémentaire comprend et/ou comprennent un ou plusieurs des additifs suivants : solvant, charge, pigment, liant, plastifiant, agent siccatif, fongicide.

22. Matériau d'adhésion et de revêtement antimicrobien selon l'une quelconque des revendications précédentes, le matériau d'adhésion et de revêtement antimicrobien étant une laque, un produit couvrant ou enduit ou une dispersion durcissable.

23. Utilisation du matériau d'adhésion et de revêtement antimicrobien selon l'une quelconque des revendications précédentes pour la fabrication et/ou l'enduction de compresses, de bandages, de produits d'incontinence, de dispositifs médicaux, de produits d'emballage, pour le revêtement de murs de bâtiments, de boîtiers ou caisses et/ou d'éléments de construction de dispositifs techniques.

24. Procédé de fabrication d'un matériau d'adhésion et de revêtement antimicrobien selon l'une des revendications 1 à 22, comprenant les étapes suivantes :
- produire une vapeur d'argent par pulvérisation ou évaporation dans un récipient sous vide,
- condenser la vapeur d'argent, de telle sorte que des particules d'argent dont la teneur en ions argent, sodium et potassium est inférieure à 5 ppm, sont formées, et
- mélanger une composante organique liquide avec les particules d'argent.

25. Procédé selon la revendication 24, dans lequel la composante organique est au moins partiellement durcie après le mélange avec les particules d'argent.

26. Procédé selon la revendication 24 ou 25, dans lequel la vapeur d'argent est condensée dans la phase gazeuse, de telle sorte que des agrégats présentant une granulométrie moyenne de 1 à 20 µm sont formés, constitués de particules primaires dont la granulométrie moyenne est comprise entre 10 et 150 nm.

27. Procédé selon la revendication 24 ou 25, dans lequel la vapeur d'argent est condensée en surface d'un liquide, de telle sorte que les particules d'argent présentent une granulométrie moyenne dans la plage de 10 à 100 nm.

28. Procédé selon l'une des revendications 24 à 27, dans lequel on utilise, en tant que liquide, la composante organique ou un pré-produit de celle-ci.

29. Procédé selon l'une des revendications 24 à 28, dans lequel l'on ajoute les particules d'argent à un pré-produit de la composante organique au lieu de la composante organique, la composante organique étant fabriquée ensuite.

30. Procédé selon l'une des revendications 24 à 29, dans lequel l'on ajoute à la composante organique 0,01 à 5 % en poids de particules d'argent.

31. Procédé selon l'une des revendications 24 à 30, dans lequel l'on mélange la composante organique avec au moins une composante organique liquide supplémentaire.

32. Procédé selon l'une des revendications 24 à 31, dans lequel la composante organique et/ou la composante organique supplémentaire comprend et/ou comprennent en tant que constituant essentiel un acrylate ou un méthacrylate.

33. Procédé selon l'une des revendications 24 à 32, dans lequel la composante organique et/ou la composante organique supplémentaire comprend et/ou comprennent une résine époxy, un uréthane, une silicone ou un cyanoacrylate en tant que constituant essentiel.

34. Procédé selon l'une des revendications 24 à 33, dans lequel des cations d'au moins l'un des métaux suivants : Au, Pt, Pd, Ir, Sn, Cu, Sb, Zn sont ajoutés à la composante organique et/ou à la composante organique supplémentaire.

35. Procédé selon la revendication 34, dans lequel les cations liés dans des dispositifs échangeurs d'ions, sont ajoutés au moins à la première composante organique sous la forme d'un complexe ou d'un sel.

36. Procédé selon la revendication 35, dans lequel le sel est un sel d'un acide carboxylique, de préférence polymérique.

37. Procédé selon l'une des revendications 24 à 36, dans lequel l'on ajoute à la composante organique et/ou à la composante organique supplémentaire un ou plusieurs des additifs suivants : solvant, charge, pigment, liant, plastifiant, agent siccatif, fongicide.
